**Europäisches Patentamt**

**European Patent Office**

**Office européen des brevets**

(19)

(11) Veröffentlichungsnummer: **0 266 544**
B1

# (12) EUROPÄISCHE PATENTSCHRIFT

(45) Veröffentlichungstag der Patentschrift:
27.12.90

(51) Int. Cl.⁵: **C07C 31/36**, C07C 29/62

(21) Anmeldenummer: **87114100.8**

(22) Anmeldetag: **26.09.87**

(54) **Verfahren zur Herstellung von Halogenalkoholen.**

(30) Priorität: **04.10.86 DE 3633886**

(43) Veröffentlichungstag der Anmeldung:
**11.05.88 Patentblatt 88/19**

(45) Bekanntmachung des Hinweises auf die Patenterteilung:
**27.12.90 Patentblatt 90/52**

(84) Benannte Vertragsstaaten:
**AT BE CH DE ES FR GB IT LI**

(56) Entgegenhaltungen:
**EP-A- 0 152 174**

**CHEMICAL ABSTRACTS, Band 98, Nr. 3, Januar 1983, Seite 473, Zusammenfassung Nr. 16274b, Columbus, Ohio, US; & JP-A-57 130 943**

(73) Patentinhaber: **BASF Aktiengesellschaft, Carl-Bosch-Strasse 38, D-6700 Ludwigshafen(DE)**

(72) Erfinder: **Becker, Rainer, Dr., Im Haseneck 22, D-6702 Bad Duerkheim(DE)**
Erfinder: **Mackenroth, Wolfgang, Dr., Seebacher Strasse 37, D-6702 Bad Duerkheim(DE)**
Erfinder: **Seufert, Walter, Dr., Laerchenweg 19, D-6720 Speyer(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Halogenalkoholen aus Diolen.

Bekanntlich entstehen bei der Umsetzung langkettiger diprimärer Diole mit Halogenwasserstoffsäuren unter anderem die Monohalogenalkohole. So erhält man nach Monatshefte 1906, 27, 411 durch Einwirkung konzentrierter Salzsäure auf Decandiol-1,10 in etwa 50 %iger Ausbeute Chlordecanol. Beim Nacharbeiten ergaben sich jedoch Probleme, vor allem bei der Reinigung, so daß in J. Chem. Soc. (1931), 1697-1701 ein Verfahren vorgeschlagen wird, welches das Wertprodukt Chlordecanol durch eine aufwendige kontinuierliche Extraktion dem Reaktionsgemisch permanent entzieht, um so die Weiterreaktion zum Decandichlorid oder zu dimeren Produkten zu vermeiden. In der Literaturstelle wird eine Ausbeuteangabe für das Chlordecanol nicht gemacht. Die Übertragung des Verfahrens auf Chlorheptanol ergibt das Produkt in 43 %iger Ausbeute. Im Falle der Herstellung von Chloroctanol erhält man zwar eine Ausbeute von 80 %, das Produkt weist jedoch nicht abtrennbare Verunreinigungen auf. Auch das Chlornonanol wurde hergestellt, jedoch findet sich auch hier keine Ausbeuteangabe. Ebenfalls ohne Angabe der Ausbeute wird die Herstellung von Chlorhexanol nach der vorstehenden Methode unter kontinuierlicher Extraktion in J. Chem. Soc. (1938), 813-815, beschrieben.

Eine eingehende Untersuchung der Umsetzung von Hexandiol mit Chlorwasserstoff ist in Monatshefte 1947, 77, 259-263 beschrieben. Hier wurde wiederum auf die umständliche kontinuierliche Extraktion verzichtet und trockener Chlorwasserstoff eingeleitet. Abhängig von den jeweiligen Bedingungen wird Chlorhexanol in einer maximalen Ausbeute von 45 % gebildet, als Nebenprodukte treten Dichlorhexan in einer maximalen Ausbeute von 35 % sowie Dichlordihexylether, Chlorhydroxydihexylether und Dihydroxydihexylether, sowie andere Produkte auf. Die analoge Umsetzung von Hexandiol mit gasförmigem HBr ist in Ber. dtsch. chem. Ges. 77 (1944), 669-675, beschrieben und führt zu überwiegendem Teil zum Dibromhexan, während Bromhexanol praktisch nicht gebildet wird. Die Synthese des Bromhexanols wird dann, wiederum unter Zuhilfenahme der kontinuierlichen Extraktion in J. Amer. Chem. Soc. (1950), 72, 5137-5139, beschrieben. Eine systematische Untersuchung der Bildung von Bromalkoholen durch Umsetzung von Diolen mit HBr und kontinuierlicher Extraktion des Reaktionsgemisches wird in Org. prep. and proc. int. 15 (1983), 63-70, beschrieben. Man erhält Ausbeuten zwischen 60 und 85 % bei 2 bis 8 % Dihalogenidgehalt und 3- bis 12-fachem HBr-Überschuß. Die in dieser Literaturstelle erwähnten Hinweise auf angebliche Herstellungen in großem Maßstab beziehen sich auf kontinuierliche Extraktionsverfahren mit Substanzmengen von weniger als 50 g. Dieses im Labor bewährte Verfahren bringt jedoch bei einer Übertragung in den technischen Maßstab große apparative Probleme mit sich, da einerseits teure Spezial-Extraktionskessel für die Reaktion und andererseits sehr große Lösungsmittelmengen oder eine zusätzliche kontinuierliche Lösungsmittelrückführung erforderlich sind. Im letzteren Fall führt das kontinuierliche Abziehen und Rückführen des Lösungsmittels in einem separaten Kessel zur Aufkonzentration geringer HHal-Mengen, die bei der Extraktion mitgerissen werden und zu Folgereaktionen (Dihalogenidbildung bzw. Etherbildung) und somit zur Produktverschlechterung führen. In Communications 1985, 1161-1163 ist eine vereinfachte Bromalkoholsynthese unter kontinuierlicher Wasserauskreisung aus dem Reaktionsgemisch beschrieben. Wenn man diese Versuche nacharbeitet, lassen sich jedoch die angegebenen Ergebnisse nicht bestätigen; im Falle der Umsetzung Butandiol zu Brombutanol erhält man lediglich Ausbeuten zwischen 50 und 60% (Literaturangabe 65%) und einen Dibromidgehalt von 2,5 bis 6% (Literaturangabe 0%) abhängig von der Geschwindigkeit des Wasserauskreisens (abhängig von der Badtemperatur). Außerdem ist auch hier die Übertragung in den technischen Maßstab wegen des erforderlichen Wasserauskreisens aufwendig und umständlich.

Die japanische Offenlegungsschrift No. 130943/1982 beschreibt ein Verfahren zur Herstellung von 1-Bromdecanol durch die Umsetzung von 1,10-Decamethylenglycol mit einer 33 % bis 40%igen wäßrigen Bromwasserstofflösung bei Reaktionstemperaturen von 60 bis 75°C. Die Ausbeute ist unbefriedigend und die Raum-Zeit-Ausbeute ungenügend.

EP-A 152 174 betrifft ein Verfahren zur Monohalogenierung von Di- und Trimethylolbenzolen. Nach den beiden Beispielen dieser Schrift können lediglich Ausbeuten von nicht mehr als 60 bzw. 54% erzielt werden.

Die erfindungsgemäß erhältlichen Halogenalkohole stellen wertvolle Zwischenprodukte dar und lassen sich bei einer großen Zahl synthetischer Anwendungen, vor allem auf dem Gebiet der Wirkstoffsynthese, einsetzen.

Der Erfindung liegt die Aufgabe zugrunde, ein Verfahren mit den Merkmalen des Oberbegriffs des Anspruchs 1 so auszugestalten, daß es die Herstellung von Halogenalkoholen in technischem Maßstab bei guter Ausbeute und hoher Selektivität erlaubt.

Diese Aufgabe wird gelöst mit einem Verfahren zur Herstellung von Halogenalkoholen der allgemeinen Formel I

Hal-X-OH (I) worin X für einen geradkettigen oder verzweigten substituierten oder unsubstituierten Alkylenrest mit mindestens 4 Kohlenstoffstomen in der Kette, der gewünschtenfalls durch ein oder mehrere Heteroatome unterbrochen ist, steht und Hal für Halogen (Fluor, Chlor, Brom, Iod) steht, das dadurch gekennzeichnet ist, daß man ein Diol der allgemeinen Formel II

HO-X-OH (II) worin X die vorstehenden Bedeutungen besitzt, mit einer wäßrigen Halogenwasserstofflösung in einem mit Wasser nicht mischbaren, unter den Reaktionsbedingungen inerten organischen Lösungsmittel bei einer Temperatur zwischen 50 und 150°C umsetzt, wobei man einen Überschuß an Halogenwasserstoff, bezogen auf das Diol der allgemeinen Formel II, von 10 bis 200 Molprozent einsetzt und ein Volumenverhältnis anorganische Phase zu organischer Phase von 1:2 bis 1:50 wählt.

Nach einer bevorzugten Ausführungsform der erfindungsgemäßen Verfahrens steht X für einen geradkettigen oder verzweigten Alkylenrest mit 4 bis 12 Kohlenstoffatomen. Besonders zweckmäßig ist der Rest X ein geradkettiger Alkylenrest mit 4 bis 12 Kohlenstoffatomen. Zweckmäßig setzt man eine wäßrige Chlorwasserstoff- oder Bromwasserstofflösung ein. Verwendet man eine Chlorwasserstofflösung, so liegt deren Konzentration zweckmäßig zwischen 18 und 36 Gew.%. Bei Verwendung einer wäßrigen Bromwasserstofflösung liegt deren Konzentration zweckmäßig zwischen 24 und 48 Gew.%.

Als organisches Lösungsmittel verwendet man vorzugsweise Kohlenwasserstofflösungsmittel, insbesondere Alkane, Cycloalkane, Aromaten, Halogenalkane oder Halogenaromaten. Besonders geeignet sind beispielsweise Hexan, Heptan, Octan oder deren Mischungen, Cyclohexan, Benzol, Toluol, die isomeren Xylole (o-, m- und p-Xylol) oder Chlorbenzole, beispielsweise Monochlorbenzol, Dichlorbenzol. Wesentlich ist, daß die organischen Lösungsmittel mit Wasser nicht mischbar sind und auch nicht selbst mit den Halogenwasserstoffsäuren reagieren. Es kommen auch im Handel erhältliche Kohlenwasserstoffgemische in Betracht, beispielsweise solche, die unter der Handelbezeichnung Skellysolve® erhältlich sind.

Beim erfindungsgemäßen Verfahren wird ein Volumenverhältnis anorganische Phase zu organischer Phase von 1:2 bis 1:50, vorzugsweise 1:5 bis 1:15, gewählt.

Die Umsetzungstemperatur beim erfindungsgemäßen Verfahren liegt zwischen 50 und 150°C, vorzugsweise zwischen 90 und 120°C.

Man setzt einen Überschuß an Halogenwasserstoff ein, der, bezogen auf das eingesetzte Diol, 10 bis 200 Mol.%, vorzugsweise 20 bis 50 Mol.%, beträgt.

Mit dem erfindungsgemäßen Verfahren lassen sich die Halogenalkohole überraschend in üblichen Kesseln durch Umsetzung der Diole mit wäßrigen Halogenwasserstoffsäuren in guten Ausbeuten und hohen Selektivitäten herstellen, ohne daß eine kontinuierliche Phasentrennung (Extraktion oder Destillation) erforderlich wäre.

Wichtig beim erfindungsgemäßen Verfahren ist eine gute Durchmischung des Zweiphasensystems, die auf übliche Weise, beispielsweise Rühren oder sonstiges Bewegen, erfolgen kann.

In den Verbindungen der allgemeinen Formel I kann der Rest X für einen geradkettigen oder verzweigten, substituierten oder unsubstituierten Alkylenrest mit mindestens 4 Kohlenstoffatomen, insbesondere 4 bis 12 Kohlenstoffatomen, in der Kette stehen. Dieser Alkylenrest kann gewünschtenfalls durch ein oder mehrere, insbesondere 1 bis 2 Heteroatome, besipielsweise Sauerstoff-, Schwefel- oder Stickstoffatome, unterbrochen sein. Beispielsweise seien folgende durch Heteroatome unterbrochene Alkylenketten aufgeführt:

$-(CH_2)_2-O-(CH_2)_2-$, $-(CH_2)_3-O-(CH_2)_3-$, $-(CH_2)_4-O-(CH_2)_4-$, $-(CH_2)_2-O-(CH_2)_2-O-(CH_2)_2-$, $-(CH_2)_2-O-(CH_2)_3-$, $-(CH_2)_2-NH-(CH_2)_2-$, $-(CH_2)_3-N(CH_3)-(CH_2)_3-$, $-(CH_2)_3-S-(CH_3)_3-$.

Bevorzugtes Heteroatom ist Sauerstoff. Im Fall von Stickstoff als Heteroatom ist der Stickstoff zusätzlich durch Wasserstoff oder einen Alkylrest, insbesondere einen $C_1-C_4$-Alkylrest substituiert. Wenn der Alkylrest substituiert ist, handelt es sich bei den Substituenten um solche, welche unter den Reaktionsbedingungen nicht mit dem Halogenwasserstoff reagieren. Beispiele für derartige Substituenten sind:

Alkyl wie Methyl, Ethyl, Propyl, Butyl, iso-Propyl, sek-Butyl, iso-Butyl, Phenyl, subst. Phenyl, Alkoxy, Carboxyl.

Als Ausgangsdiol der allgemeinen Formel II kommen besonders bevorzugt in Frage 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol sowie 1,12-Dodecandiol. Die Diole der allgemeinen Formel II sind entweder bekannt oder nach an sich bekannten Arbeitsweisen erhältlich.

Die Erfindung wird durch die nachstehenden Beispiele weiter erläutert.

Beispiel 1

Herstellung von 8-Bromoctanol-1

Man rührte 235 kg Octandiol-1,8 in einem 6 m³ Kessel mit 3 m³ Toluol und 440 kg HBr (48 %ig) 40 Stunden lang unter Rückfluß. Anschließend trennte man die wäßrige Phase ab. Die Toluolphase wurde mit 500 l Wasser, anschließend 500 l Sodalösung (10 %) und dann wieder 500 l Wasser gut gewaschen. Eine entnommene Probe zeigte nach dem Einengen das Vorliegen von 92 % Bromoctanol neben 1,9 % Dibromoctan und 2,0 % unumgesetztem Octandiol. Nach dem Abdestillieren des Toluols im Reaktionskessel und einer Sambay-Destillation des verbleibenden Rückstands betrug die Ausbeute 254 kg (76 %).

Beispiel 2

Herstellung von 6-Chlorhexanol-1

In einem 250 l Kessel wurden 14,16 kg Hexandiol-1,6 mit 150 l Toluol und 30 l wäßriger HCl (36 %ig) versetzt und 36 Stunden am Rückfluß gehalten. Die Aufarbeitung erfolgte wie im Beispiel 1 beschrieben, die Reinigung wurde über eine Destillation erzielt (Kp $_{3mbar}$ = 83°C). Die Ausbeute betrug 13,3 kg (82 %) 6-Chlorhexanol-1.

**Patentansprüche**

1. Verfahren zur Herstellung von Halogenalkoholen der allgemeinen Formel I
Hal-X-OH (I)
worin X für einen geradkettigen oder verzweigten, substituierten oder unsubstituierten Alkylenrest mit mindestens 4 Kohlenstoffatomen in der Kette, der gewünschtenfalls durch ein oder mehrere Heteroatome unterbrochen ist, steht und Hal für Halogen steht, dadurch gekennzeichnet, daß man ein Diol der allgemeinen Formel II

HO-X-OH(II) worin X die vorstehenden Bedeutungen besitzt, mit einer wäßrigen Halogenwasserstofflösung in einem mit Wasser nicht mischbaren, unter den Reaktionsbedingungen inerten organischen Lösungsmittel bei einer Temperatur zwischen 50 und 150°C umsetzt, wobei man einen Überschuß an Halogenwasserstoff, bezogen auf das Diol der allgemeinen Formel II, von 10 bis 200 Molprozent einsetzt und ein Volumenverhältnis anorganische Phase zu organischer Phase von 1:2 bis 1:50 wählt.

2. Verfahren nach Anspruch 1, dadurch gekennzeichnet, daß X für einen geradkettigen oder verzweigten Alkylenrest mit 4 bis 12 Kohlenstoffatomen steht.

3. Verfahren nach einem der Ansprüche 1 oder 2, dadurch gekennzeichnet, daß man eine wäßrige Chlorwasserstoff- oder Bromwasserstofflösung einsetzt.

4. Verfahren nach einem der Ansprüche 1 bis 3, dadurch gekennzeichnet, daß man ein geradkettiges diprimäres $C_4$-$C_{12}$-Diol einsetzt.

5. Verfahren nach einem der Ansprüche 1 bis 4, dadurch gekennzeichnet, daß man als organisches Lösungsmittel Kohlenwasserstoffe oder halogenierte Kohlenwasserstoffe, insbesondere Alkane, Cycloalkane, Aromaten, Halogenalkane, Halogenalkene oder Halogenaromaten einsetzt.

6. Verfahren nach Anspruch 5, dadurch gekennzeichnet, daß man als organisches Lösungsmittel Hexan, Heptan, Octan, oder deren Mischungen, Cyclohexan, Benzol, Toluol, Xylole oder Chlorbenzole einsetzt.

7. Vefahren nach einem der Ansprüche 1 bis 6, dadurch gekennzeichnet, daß man ein Volumenverhältnis anorganische Phase zu organischer Phase von 1:5 bis 1:15 wählt.

## Revendications

1. Procédé de préparation d'alcools halogénés répondant à la formule générale I

Hal-X-OH     (I)

dans laquelle X représente un radical alkylène linéaire ou ramifié, substitué ou non substitué, dont la chaîne comporte au moins 4 atomes de carbone et est, si cela se révèle souhaitable, interrompue par un ou plusieurs hétéroatomes et Hal représente un halogène, caractérisé en ce que l'on fait réagir un diol de la formule générale II

HO-X-OH     (II)

dans laquelle X représente les signification qui lui ont été attribuées ci-dessus, avec une solution aqueuse d'un acide halogénhydrique, à une température variant de 50 à 150° C, dans un solvant organique, inerte dans les conditions réactionnelles et non miscible à l'eau, et l'on choisit un excès d'acide halogénhydrique de 10 à 200 % molaires, par rapport au diol de la formule générale II, ainsi qu'un rapport volumique de la phase inorganique à la phase organique de 1:2 à 1:50.

2. Procédé suivant la revendication 1, caractérisé en ce que X représente un radical alkylène, linéaire ou ramifié, qui comporte de 4 à 12 atomes de carbone.

3. Procédé suivant l'une quelconque des revendications 1 et 2, caractérisé en ce que l'on utilise une solution aqueuse d'acide chlorhydrique ou d'acide bromhydrique.

4. Procédé suivant l'une quelconque des revendications 1 à 3, caractérisé en ce que l'on utilise un diol en $C_4$ à $C_{12}$, diprimaire, linéaire.

5. Procédé suivant l'une quelconque des revendications 1 à 4, caractérisé en ce que l'on utilise, à titre de solvant organique, des hydrocarbures ou des hydrocarbures halogénés, plus particulièrement des alcanes, des cycloalcanes, des composés aromatiques, des halogénalcanes, des halogénalcènes ou des composés aromatiques halogénés.

6. Procédé suivant la revendication 5, caractérisé en ce que l'on utilise, à titre de solvant organique, l'hexane, l'heptane, l'octane ou leurs mélanges, le cyclohexane, le benzène, le toluène, des xylènes ou des chlorobenzènes.

7. Procédé suivant l'une quelconque des revendications 1 à 6, caractérisé en ce que l'on choisit le rapport volumique de la phase inorganique à la phase organique de 1:5 à 1:15.

## Claims

1. A Process for preparing a haloalcohol of the formula I

Hal-X-OH (I)

where X is straight-chain or branched, substituted or unsubstituted alkylene of 4 or more carbon atoms in the chain, which may be interrupted by one or more heteroatoms, and Hal is halogen, which comprises reacting a diol of the formula II

HO-X-OH (II)

where X has the above meanings, with an aqueous hydrogen halide solution in a water-immiscible organic solvent which is inert under the reaction conditions, at from 50 to 150°C, using an excess of hydrogen halide, based on the diol of the formula II, cf from 10 to 200 mole percent and setting a volume ratio of inorganic phase: organic phase of from 1:2 to 1:50.

2. A Process as claimed in claim 1, wherein X is straight-chain or branched alkylene of 4 to 12 carbon atoms.

3. A process as claimed in either of claims 1 and 2, wherein aqueous hydrogen chloride or hydrogen bromide solution is used.

4. A process as claimed in any of claims 1 to 3, wherein a straight-chain diprimary $C_4$–$C_{12}$-diol is used.

5. A process as claimed in any of claims 1 to 4, wherein the organic solvent used is a hydrocarbon or halogenated hydrocarbon, in particular an alkane, cycloalkane, aromatic, haloalkane, haloalkene or haloaromatic.

6. A process as claimed in claim 5, wherein the organic solvent used is hexane, heptane, octane or a mixture thereof, cyclohexane, benzene, toluene, xylenes or chlorobenzenes.

7. A process as claimed in any of claims 1 to 6, wherein the volume ratio of inorganic phase: organic phase is selected to range from 1:5 to 1:15.